# EUROPEAN PATENT SPECIFICATION

(11) **EP 4 284 331 B1**
(45) Date of publication and mention of the grant of the patent: **02.12.2026**
(21) Application number: 22702676.2
(22) Date of filing: 26.01.2022
(51) Int. Cl.: A61K 8/9706, A61Q 19/00, A61K 8/99, A61K 36/02

(54) **MICROALGAE CALCAREOUS COMPOSITIONS AND USES THEREOF**
KALKHALTIGE MIKROALGENZUSAMMENSETZUNGEN UND DEREN VERWENDUNGEN
COMPOSITIONS CALCAIRES DE MICROALGUES ET LEURS UTILISATIONS

(30) Priority: 26.01.2021 EP 21305095
(43) Date of publication of application: 06.12.2023
(73) Proprietor: Miyoshi Europe, 69800 Saint-Priest (FR)
(72) Inventor: TAKAHASHI, Tetsuya, 69800 Saint-Priest (FR)
(74) Representative: Icosa
(86) International application number: PCT/EP2022/051788
(87) International publication number: WO 2022/162019

(56) References cited:
- WO-A1-2008/157629
- US-A1- 2019 225 931
- TANGEN KARL ET AL: "Thoracosphaera heimii (Lohmann) Kamptner is a dinophyte: Observations on its morphology and life cycle", MARINE MICROPALEONTOLOGY, vol. 7, no. 3, 1 June 1982 (1982-06-01), AMSTERDAM, NL, pages 193 - 212, XP055822359, ISSN: 0377-8398, DOI: 10.1016/0377-8398(82)90002-0
- WENDLER JENS ET AL: "Palaeoecology of calcareous dinoflagellate cysts in the mid-Cenomanian Boreal Realm: implications for the reconstruction of palaeoceanography of the NW European shelf sea", CRETACEOUS RESEARCH, ACADEMIC PRESS, AMSTERDAM, NL, vol. 23, no. 2, 18 September 2002 (2002-09-18), pages 213 - 229, XP029576406, ISSN: 0195-6671, DOI: 10.1006/CRES.2002.0311

## Description

### FIELD OF INVENTION

The present invention relates to the field of inorganic compositions, namely microalgal origin compositions suitable for cosmetic, personal care, pharmaceutical and food applications.

### BACKGROUND OF INVENTION

Microplastics beads have typically been used in cosmetics and personal care products, typically scrubbing face/body creams, shower gels and hand cleaners or coloring beads in toothpastes. The most common polymer used in such applications is polyethylene and is applied in a variety of cosmetic products,
Microplastic hollow beads may also be used as a carrier for active pharmaceutical ingredients, where the active substances are placed in the hollow interior and slowly diffuse through the body. Such microbeads are widely used in medical and biological applications as carriers, such as for example drug delivery systems.

However, due to their polymeric nature such particles are not biodegradable, and because of their size they are easy to be dispersed throughout the globe and bioaccumulate via the food chain. Consequently, the widespread use of such microplastics is an environmental and health challenge.

As evidenced by the Final report of the European strategy on plastic waste in the environment (European Commission, DG ENV, 28 November 2012) the use of microplastics should be limited or carefully studied before placing microplastics on the market if not phased-out from the market. Thus, there is a need to develop alternative carriers for cosmetic, personal care, and pharmaceuticals that are not harmful to the environment and to the human health.

The cosmetic field is permanently looking for innovations, in particular, new active substances and oceanic resources could be of great help for this purpose. Although macroalgae are already widely exploited in the cosmetic industry, it is not the case for microalgae. Limited microalgae-based cosmetic or pharmaceutical ingredients have been reported such as moisturizing proteins, anti-aging active ingredients, mycosporine-like amino acids as sunscreens, melanin inhibitors, preservatives and dyes (Couteau and Coiffard, Microalgae in Health and Disease Prevention, Academic Press, 2018, Pages 317-323).

The French patent FR3054128 discloses the cosmetic use of coccolithoforid (Haptophyta branch, class Prymnesiophyceae or Coccolithophyceae) and in particular of their calcareous exoskeletons (coccoliths) as sunscreen and anti-inflammatory agents. However, the Haptophyta coccoliths are an assembly of building units, also known as placoliths. Thus, Haptophyta coccoliths are mechanically instable and do not maintain their three-dimensional structure upon drying and/or mixing with other ingredients. Therefore, such coccoliths cannot be considered as an efficient replacement for microplastic hollow beads.

Thoracosphaera heimii (Lohmann) are known dinophyte with spherical shape and a cell diameter in the range 13-18 micron (TANGEN KARL ET AL: "Thoracosphaera heimii (Lohmann) Kamptner is a dinophyte: Observations on its morphology and life cycle", 1 June 1982, MARINE MICROPALEONTOLOGY, vol. 7, no. 3, pages 193-212).

The Applicant has surprisingly found that a composition of calcareous exoskeletons of Dinoflagellate microalgae, as defined in the present specification, can address the problems discussed above and can be used in the field of cosmetic, pharmaceutical and food products.

### SUMMARY

The invention relates to a process for producing a cosmetic, a personal care, a pharmaceutical or a nutraceutical composition, the process comprising:
a) cultivating *Thoracosphaera heimii* microalgae cells, then
b) recovering the cultured microalgae, then
c) washing the recovered *Thoracosphaera heimii* cell culture with an aqueous solution, then rejecting the aqueous solution and recovering solid phase consisting of the *Thoracosphaera heimii* calcispheres, then
d) drying the calcispheres, and then
e) mixing the dried calcispheres of step (d) with at least one cosmetically, personal care, pharmaceutically or nutraceutically acceptable ingredient.

In one embodiment, the aqueous solution is 100% water.

In on embodiment, the dried calcispheres of step (d) are calcinated prior to the mixing step (e).

In one embodiment, the process of the invention is for producing a cosmetic composition and the step (e) consists in mixing the dried calcispheres of step (d) with at least one cosmetically acceptable ingredient.

The invention also relates to the use of *Thoracosphaera heimii* microalgae as a cosmetically or nutraceutically acceptable ingredient.

In one embodiment, *Thoracosphaera heimii* is used as a whole cell.

In one embodiment, *Thoracosphaera heimii* is used as calcispheres.

In one embodiment, *Thoracosphaera heimii* is used as a cosmetically acceptable excipient.

In one embodiment, *Thoracosphaera heimii* is used as a carrier for cosmetically, pharmaceutically or nutraceutically acceptable active ingredient.

The invention also relates to a cosmetic, personal care, pharmaceutical or nutraceutical composition comprising spherical *Thoracosphaera heimii* calcispheres of less than 15 µm average diameter and of sphere wall thickness of less than 1 µm, in association with at least one cosmetically, pharmaceutically or nutraceutically acceptable ingredient.

In one embodiment, the composition of the invention comprises 0.1 % to 100 % of the spherical *Thoracosphaera heimii* calcispheres, in weight of the total cosmetic, a personal care, a pharmaceutical or a nutraceutical composition.

### DEFINITIONS

In the present invention, the following terms have the following meanings:
- **"Calcification"** refers to the biochemical process of calcium carbonate formation by a range of intracellular and/or extracellular processes. The process of such biogenic calcification occurs widely in nature. Both prokaryotes and eukaryotes may contribute to up to 25 % of biogenic calcification, while coral reefs and free-living foraminifera (protozoa) generate most of the rest. The calcification process is a biochemical reaction based on the following overall reaction.

   2HCO₃⁻ + Ca²+↔ CaCO₃ +CO₂+ H₂O

   The obtained biomineral calcium carbonate is in the form of calcite presenting a rhombohedral crystal structure.
- **"Calcispheres"** designates hollow, typically spherical, calcareous exoskeleton structures from calcareous Dinoflagellate *Thoracosphaera heimii* microalgae, also referred as *Thoracosphaera heimii.* In one embodiment, the calcisphere wall comprises calcite elements with their c-axes tangential to the wall. In one embodiment, the calcisphere wall comprises calcite elements with their c-axes perpendicular to the wall. Typically, calcispheres present a mean diameter from 5 to 50 µm.
- **"Dinoflagellates"** are single-celled eukaryotic microalgae constituting the phylum Dinoflagellata. Usually Dinoflagellates are mostly marine plankton, but they also are common in freshwater habitats. The present invention refers to calcareous dinoflagellates, that are capable of forming calcareous structures, namely calcispheres. The calcareous dinoflagellates are selected from the order of Peridiniales and/or Thoracosphaerales. Such calcareous structures may also be designated as dinocysts or calcispheres.
- **"Cosmetically, pharmaceutically, or nutraceutically acceptable ingredients"** refer to the respective excipients or cosmetically/therapeutically/nutraceutically active compounds that are appropriate for such uses and do not provoke any side effects such as toxicity, irritation, inflammation or allergic response. Such excipients may be selected from bulking agents, fillers, diluents that may facilitate the production, application or absorption of a cosmetical composition, dyes, coatings, dispersants, anti-tacking agents, preservatives or flavors. In the context of the present invention a cosmetically acceptable excipients also refer to personal-care product acceptable ingredients. The present composition may be of particular use in the formulation of cosmetically/therapeutically/ nutraceutically active compounds previously disclosed in the art. **Pharmaceutically or therapeutically active compound** refers to an active principle to be optionally in association with a pharmaceutically acceptable vehicle or excipient leading to a pharmaceutical composition. A pharmaceutical composition is for therapeutic use, and relates to health. Especially, a pharmaceutical composition may be indicated for treating or preventing a disease. According to the invention, the term "treating a disease" refers to reducing or alleviating at least one adverse effect or symptom of a disease, disorder or condition associated with a deficiency in an organ, tissue or cell function. The expression "Preventing a disease" or "Inhibiting the development of a disease" refers to preventing or avoiding the occurrence of symptom. **Cosmetically active compound refers to** an active principle, optionally in association with a cosmetically acceptable vehicle or excipient, leading to a cosmetic composition. The use of a cosmetic composition does not encompass therapeutic uses, and relates to well-being and beauty. **Nutraceutically active** compound refers to a product isolated or purified from comestibles (foodstuff). A nutraceutical is demonstrated to have a physiological benefit or provide protection against physiological disorder or discomfort without encompassing therapeutic uses/applications.
- **"exoskeleton"** refers to an external structure containing rigid and resistant components that fulfill a set of functional roles in microalgae including protection, excretion, sensing, support, feeding and acting as a barrier during vegetative and/or dormant phases.
- **"Haptophytes" or "Coccolithophores"** are calcifying microalgae belonging to the Haptophyceae. Several haptophytes have been reported to present a calcareous exoskeleton structure. Depending on the species, and the haptophyte growth phase, such microalgae may present holococcoliths (small cuboid crystallite-based discontinuous structures), heterococcoliths (complex scale-based discontinuous structures). Spheric holococcoliths and heterococcoliths may also be indexed as coccospheres, such as for example the coccospheres of *Emiliania huxleyi.* Coccospheres easily decompose to their coccolith building units (coccoliths or placoliths) (Van der Wal et al. Production and downward flux of organic matter and calcite in a North Sea bloom of the coccolithophore Emiliania huxleyi, Marine Ecology progress series, 1995, Vol. 126: 247-265).
   Whereas calcispheres, as defined above, possess a continuous calcareous wall, coccospheres (holococcoliths or heterococcoliths of Haplophytes) are composite structures formed of numerous building units (coccoliths or placoliths). In one embodiment, the present use, method and/or composition does not encompass Haptophyte microalgae and/or their holococcolith or heterococcolith exoskeletons.
- **"Microalgae"** refers to microscopic predominantly unicellular algae, typically found in freshwater and marine systems and from 2 to 65 µm in diameter. They are unicellular species which exist individually, or in chains or groups as opposed to macroalgae or seaweeds that are pluricellular organisms. Microalgae encompass the taxa of Dinoflagellates and Haptophytes whose members may present calcareous exoskeletons.
- **"Peridiniales"** also indexed as Thoracosphearace, is a taxon of calcareous Dinoflagellates. Peridiniales encompass the calcisphere-producting species *Bicarinellum tricarinelloides, Calcicarpinum bivalvurn, Calcigonellum infula, Calciodinellum albatrosianum, Calciodinellum albatrosianum, Calciodinellum elongatum, Calciodinellum levantinum, Calciodinellum operosum, Calciperidinium asymmetricum, Caracomia arctica, Ensiculifera carinata, Follisdinellum splendidum, Fuettererella cf. tesserula, Lebessphaera urania, Leonella granifera, Melodomuncula berlinensis, Pentadinellum oblatum, Pernambugia tuberosa, Praecalcigonellum schizosaeptum, Scrippsiella crystallina, Scrippsiella lachrymose, Scrippiella precaria, Scripssiella ramonii, Scrippsiella regalis, Scrippsiella rotunda, Scrippsiella trifida, Scrippsiella triquetracapitata, Scrippsietla trochoidea,* and *Thoracosphaera heimii.*
   The calcareous Dinoflagellate microalgae according to the invention are of the *Thoracosphaera heimii* species.
- **"Photobioreactor"** refers to any manufactured device or system that supports a biologically active environment using a light source to cultivate phototrophic microorganisms such as calcareous Dinoflagellates. In an exopolysaccharide secretion study *Scripsiella* and *Thoracosphaera* species were successfully culture in photobioreactor conditions (Gaignard et al. Screening of marine microalgae: investigation of new exopolysaccharide producers, Algal Research 2019, supplementary table1 part 5). *Thoracosphaera heimii, Leonella granifera* and *Calciodinellum levantinum* have also been cultured with success by Meier *et al.* (Meier et al. Evolution of different life-cycle strategies in oceanic calcareous dinoflagellates, European Journal of Phycology, 2007, 42(1):81-89).
- **"sphericity index"** Sphericity is a three-dimensional measurement of the divergence of a shape from a perfect sphere. Sphericity is calculated as 4πA/P², where P is the perimeter and A is the area of the particle projection. A perfect sphere has a sphericity of unity (scored as a 1 on the scale), while other shapes have a sphericity index of less than 1.
- **"Thoracosphaera heimii"** designates the calcareous Dinoflagellate *Thoracosphaera heimii* (Lohmann 1920) Kamptner 1944. *T. heimmii* is one of the most common species, especially in open ocean environments. Samples of *T. heimmii* have also been collected in the Mediterranean Sea (Meier et al. Evolution of different life-cycle strategies in oceanic calcareous dinoflagellates, European Journal of Phycology, 2007, 42(1):81-89). The *T. heimmii* calcispheres are spherical. The calcisphere wall may present one sub-circular opening of less than 5 µm diameter and further submicron perforations of less than 0.2 µm diameter. The calcite elements of the calcisphere wall are interlocked and their c-axes are tangential to the calcisphere wall.

### DETAILED DESCRIPTION

The invention relates to a process for producing a cosmetic, a pharmaceutical or a nutraceutical composition, the process comprising:
a) cultivating calcareous Dinoflagellate *Thoracosphaera heimii* microalgae cells, also referred as *Thoracosphaera heimii,* then
b) recovering the cultured microalgae, then
c) washing the recovered *Thoracosphaera heimii* cell culture, typically with an aqueous solution, then rejecting the aqueous solution and recovering solid phase consisting of the *Thoracosphaera heimii* calcispheres, then
d) drying the calcispheres, and then
e) mixing the dried calcispheres of step (d) with at least one cosmetically, pharmaceutically or nutraceutically acceptable ingredient.

The step (a) comprising culturing *Thoracosphaera heimii* microalgae cells as defined above, can be carried out according to any methods known in the art. Typically, microalgae can be cultured in liquid media. The culture can be contained within a bioreactor or in sea-water pools. Microalgae can also be cultured in photobioreactors that contain a fixed carbon source and allow light to strike the cells. Exposure of microalgae cells to light, even in the presence of a fixed carbon source that the cells transport and utilize, can accelerate growth compared to culturing cells in the dark.

In one particular embodiment, *Thoracosphaera heimii* microalgae cells are cultivated in a photobioreactor. Such bioreactors can be exposed to one or more light sources to provide microalgae with a light signal. A light signal can be provided via light directed to a surface of the bioreactor by a light source. Preferably the light source provides an intensity that is sufficient for the cells to grow, but not so intense as to cause oxidative damage. In some instances, a light source has a wavelength range that mimics or approximately mimics the range of the sun. In other instances, a different wavelength range is used. Bioreactors can be placed outdoors or in a greenhouse or other facility that allows sunlight to strike the surface. The culture medium can be illuminated by natural or artificial lighting, in particular by the use of neon or LED lamps, with photoperiods ranging from 12h day / 12h night to 24h day / 0h night.

Photobioreactors may be closed, as in the instance of a polyethylene bag or Erlenmeyer flask, or may be open to the environment, as in the instance of an outdoor pond. In one particular embodiment, the *Thoracosphaera heimii* microalgae are cultivated in air-lift method bioreactors wherein air is moved within a system in order to circulate water where microalgae are growing. The culture is grown in transparent tubes that lie horizontally on the ground and are connected by a network of pipes. Air is passed through the tube such that air escapes from the end that rests inside the reactor that contains the culture and creates an effect like stirring.

In the context of the present invention, the term "cultivate" refers to the intentional promoting of growth (cell size, cellular contents, and/or cellular activity) and/or propagation. Examples of microalgae cultivation conditions include the use of a defined medium (with known characteristics such as pH, ionic strength, and carbon source), specified temperature, oxygen tension, carbon dioxide levels, and growth in a bioreactor. Microalgal culture media typically contains components such as a fixed nitrogen source, trace elements, optionally a buffer for pH maintenance, and phosphate. Other components can include a fixed carbon source such as acetate or glucose, and salts such as sodium chloride, particularly for seawater microalgae. Examples of trace elements include zinc, boron, cobalt, copper, manganese, and molybdenum. A typical example of such microalgal culture media is K medium consisting of seawater enriched with NaNO₃, Na₂-glycerophosphate·5H₂O, NH₄Cl, Tris-base (pH 7.2); a Microelement stock solution of Fe Na EDTA, Na₂EDTA•2H₂O, CuSO₄•5H₂O, MnCl₂•4H₂O, ZnSO₄•7H₂O, Na₂MoO₄•2H₂O, H₂SeO₃, FeCl₃ •6H₂O, CoCl₂ •6H₂O; and a Vitamin stock solution comprising Biotin (Vitamin H) Thiamine HCl (Vitamin B1) Cyanocobalamin (Vitamin B12).

The choice of such conditions is well known in the art. For instance, any type of enriched seawater medium such as K medium, f/2 medium, PCR-S11 Medium, CHU-11 Medium, ASN-III Medium or Walne's Medium is suitable for the step (a) of the present method. Indicatively, the culture of *T. heimii* using K medium is reported by Meier *et al 2007.*

Microalgae cultures can also be subjected to mixing using devices such as spinning blades and impellers, rocking of a culture, stir bars, or infusion of pressurized gas.

The cell culture temperature and duration can be adjusted by one of the ordinary skills in the art, especially in view of the culture volume and aimed culture yield. Typically, the culture is maintained for at least one week, at least two weeks or at least three weeks. Typically, the culture medium temperature ranges from 10 to 30°C or from 10°C to 20°C. In one particular embodiment, the culture medium temperature ranges from 10°C to 15°C.

Recovering the microalgae cells according to step (b) may be done with any known technique in the art. For instance, the microalgae cells of the invention can be harvested, or otherwise recovered, by any convenient means, such as for example by filtering or sieving the culture medium containing the microalgae or by centrifuging the culture medium containing the microalgae. In an alternative embodiment, the microalgae are recovered by precipitation or flocculation by adding flocculant agents such as aluminum sulphate or ferric chloride.

The biomass can then be washed according to step (c) with a washing solution, preferably and aqueous solution in order to remove the culture medium, the microalgae organic material and debris. In one embodiment, the washing solution is 100% water. According to one variant the water is distilled water. According to another variant the water is isotonic or hypertonic. According to an alternative embodiment the washing solution comprises up to 30% of an alcohol such as ethanol or propanol in an amount ranging from 1% to 30% in volume relative to the washing solution. According to an optional embodiment, the washing solution comprises a base such as hydroxides, carbonates and bicarbonates of lithium, sodium, potassium, calcium, and mixtures thereof, typically the base is potassium hydroxide.

The washing step (c) may be implemented by any convenient means known in the art such as for example dispersing the recovered microalgae cells of step (b) into the washing solution and then recovering the solid phase of microalgae cells as indicatively detailed above, or pouring the washing solution through the recovered microalgae cells' solid phase.

Optionally, the washing step further comprises washing the recovered microalgae with a solution comprising proteases, cellulases, hemicellulases and/or pectinases. Such optional treatment can be carried out sequentially or simultaneously to the washing with the aqueous washing solution.

The recovered and washed microalgae cell solid phase essentially consists of microalgae calcispheres. In one embodiment, the obtained composition in step (c) comprises at least 85% w/w, at least 90% w/w or, at least 95% w/w or at least 98% w/w of calcispheres, in weight relative to the dry weight of said recovered and washed solid phase.

The recovered and washed microalgae cell solid phase is then dried according to step (d). Drying may be carried-out with any convenient means known in the art such as for example oven drying, microwave drying, vacuum drying, solar drying, infrared drying, fluidized-bed drying or lyophilization.

The dried solid phase essentially consists of microalgae calcispheres. In one embodiment, the obtained composition in step (d) comprises at least 85% w/w, at least 90% w/w or, at least 95% w/w or at least 98% w/w of calcispheres, in weight relative to the weight of the total composition.

The obtained calcispheres are hollow and present a spherical shape with a sphere wall thickness ranging from 0.5 µm to 1.5 µm, preferably less than 1.0 µm. In one embodiment, the *Thoracosphaera heimii* calcispheres present a sphericity index of at least 0.85, at least 0.90, typically at least 0.95 or more.

In one embodiment, the implementation of steps (a) to (d) does not affect the structure of the calcispheres, in particular the spherical shape of the calcispheres is maintained throughout steps (a) to (d).

In one embodiment, the calcisphere wall may present one sub-circular opening of less than 8 µm diameter and further submicron perforations of less than 0.5 µm diameter. In one embodiment, the calcisphere wall may present one sub-circular opening of less than 5 µm diameter and further submicron perforations of less than 0.2 µm diameter. In one embodiment, the calcite elements of the calcisphere wall are interlocked and their c-axes are tangential to the calcisphere wall.

The particle size and size distribution may be measured by Scanning Electron Microscopy (SEM), light (typically laser) scattering/diffraction or the sieving particle composition. Typically, the size distribution of the present calcisphere composition is measured by laser scattering.

Scanning electron microscopy (SEM) is an analytical instrument that uses a focused beam of high-energy electrons to generate a variety of signals at the surface of solid specimens. The signals reveal information about the sample including external morphology (texture), and orientation of materials making up the sample. The particle size of a particle is considered to be the largest linear dimension of each calcisphere particle, whereby the largest linear dimension of a particle is measured as the largest straight-line distance between two points on the periphery of the particle as observed by electron microscopy of the particle. In the case of the spherical microalgae particles, the average size corresponds to the average diameter. In one embodiment, the SEM images are obtained using Tescan VEGA3 microscope. Suitable software for automatically performing the SEM image analysis is generally known in the art.

In one preferred embodiment, the size distribution is measured by laser scattering. In one embodiment, the size distribution is measured with a Mastersizer 2000 device from Malvern. The average diameter of the calcisphere particles can be measured using a particle diameter distribution obtained using a laser diffraction/scattering method.

The obtained calcisphere composition presents an average diameter of less than 20 µm, preferably less than 15 µm. In one embodiment, the obtained calcispheres present a homogeneous size distribution, typically a monomodal distribution, preferably at least 80 % of the calcispheres present a diameter of less than 15 µm. In one embodiment, at least 85 % of the calcispheres present a diameter of less than 15 µm. In one embodiment, at least 90 % of the calcispheres present a diameter of less than 15 µm. The particle size distributions according to the above embodiments were calculated by laser scattering.

Optionally, the dried composition may further be calcinated in order to remove any remaining organic material traces.

The dried solid phase of step (d) is then mixed with at least one cosmetically acceptable ingredient in order to obtain a cosmetic composition or a personal care product.

Cosmetically acceptable ingredient may be selected from any cosmetically acceptable ingredient known in the art such as for example abrasive agents, absorbents, anticaking agents, anticorrosive agents, cosmetically acceptable antimicrobial agents, active agent carrier, antifoaming agents, antioxidants, antiperspirants, antiplaque agents, antiseborrhoeic agents, antistatic agents, astringents, binding agents, bleaching agents, buffering agents, bulking agents, chelating agents, cleansing agents, cosmetic colorants, denaturants, deodorants, depilatories, detangling agents, emollients, emulsifying agents, emulsion stabilizers, film formers, flavoring agents, foam boosting agents, foaming agents, gel forming agents, hair conditioning, hair dyeing agents, hair fixing agents, hair straightening agents, hair waving agents, humectants, hydrotropics, keratolytics, masking agents, moisturizing agents, nail conditioning agents, opacifying agents, oral care agents, oxidizing agents, pearlescent agents, perfuming agents, plasticizers, preservatives, propellants, reducing agents, refatting agents, refreshing agents, skin conditioning, skin protecting agents, smoothing agents, solvents, soothing agents, stabilising agents, surfactants, tanning agents, tonic, UV absorbers, UV filters and/or viscosity controlling agents.

The cosmetic composition may be selected for example from solid such as powdered foundation, liquid make-up compositions, beauty creams and/or anti-aging creams. According to such embodiment and without willing to be bound by a theory, the obtained calcispheres are advantageous due to their mattifying effect, skin-texture improving effect, skin-blurring effect and/or or their capacity to act as a vehicle for the cosmetic ingredients.

In one embodiment, the cosmetic composition is a skin care composition. In one embodiment, the cosmetic composition is a make-up composition.

The personal care product may be selected for example from sunscreen creams or lotions, toothpastes, antiperspirant compositions, shower gels, shampoos, exfoliating compositions, feminine care compositions or baby care compositions. According to one advantageous aspect of the invention, the obtained calcispheres can replace talc in cosmetic or personal care compositions that contain talc such as make-up compositions, feminine care compositions or baby care compositions, such as baby-powder compositions.

Alternatively, the dried solid phase of step (d) is then mixed with at least one pharmaceutically acceptable ingredient in order to obtain a pharmaceutical composition.

Pharmaceutically acceptable ingredient may be selected from any pharmaceutically acceptable excipient known in the art such as for example absorbents, anticaking agents, anticorrosive agents, plant extracts, essential oils, antioxidants, binding agents, buffering agents, bulking agents, chelating agents, emollients, emulsifying agents, emulsion stabilizers, film formers, flavoring agents, humectants, hydrotropics, keratolytics, opacifying agents, oxidizing agents, perfuming agents, plasticizers, preservatives, propellants, reducing agents, skin conditioning, skin protecting agents, smoothing agents, solvents, soothing agents, stabilizing agents, surfactants, and/or viscosity controlling agents.

Pharmaceutically acceptable ingredient may be selected from any pharmaceutically or therapeutically active compound known in the art such as for example antibacterial agents, anti-antiviral agents, antifungal agents, anti-inflammatory agents, cytotoxic agents, and/or any other known pharmaceutical agent suitable for a therapeutic use. In one embodiment, the pharmaceutical composition is for topical external use. In one embodiment, the pharmaceutical composition is an oral pharmaceutical composition.

Alternatively, the dried solid phase of step (d) is then mixed with at least one nutraceutically acceptable ingredient in order to obtain a nutraceutical composition.

Nutraceutically acceptable ingredients may be selected from any nutraceutically acceptable excipient known in the art such as for example absorbents, fibers selected from oligosaccharides and polysaccharides, probiotics, plant oils, fish oils, plant extracts, essential oils, vitamins, oligominerals, antioxidants, bulking agents, binding agents, anticaking agents, buffering agents, bulking agents, chelating agents, emulsifying agents, emulsion stabilizers, flavoring agents, humectants, opacifying agents, oxidizing agents, perfuming agents, plasticizers, preservatives, solvents, soothing agents, stabilizing agents, surfactants, and/or viscosity controlling agents.

In one embodiment, the cosmetic, personal care, pharmaceutical or nutraceutical composition consists of dried calcispheres such as the calcispheres of step (d) defined above. In one embodiment, the cosmetic, personal care, pharmaceutical or nutraceutical composition comprises from 0.1 % to 99% of dried calcispheres, in weight of the total cosmetic, personal care, pharmaceutical or nutraceutical composition. In one embodiment, the cosmetic, personal care, pharmaceutical or nutraceutical composition comprises from 20 % to 90% of dried calcispheres, in weight of the total cosmetic, personal care, pharmaceutical or nutraceutical composition. In one embodiment, the cosmetic, personal care, pharmaceutical or nutraceutical composition comprises from 30 % to 85% of dried calcispheres, in weight of the total cosmetic, personal care, pharmaceutical or nutraceutical composition. In one embodiment, the cosmetic, personal care, pharmaceutical or nutraceutical composition comprises from 30 % to 70% of dried calcispheres, in weight of the total cosmetic, personal care, pharmaceutical or nutraceutical composition. In one embodiment, the cosmetic, personal care, pharmaceutical or nutraceutical composition comprises from 40 % to 60% of dried calcispheres, in weight of the total cosmetic, personal care, pharmaceutical or nutraceutical composition.

In one embodiment, the cosmetic, personal care, pharmaceutical or nutraceutical composition comprises from 0.1 % to 15% of dried calcispheres, in weight of the total cosmetic, personal care, pharmaceutical or nutraceutical composition. In one embodiment, the obtained cosmetic, personal care, pharmaceutical or nutraceutical composition comprises from 1 % to 10 % of dried calcispheres, in weight of the total cosmetic, personal care, pharmaceutical or nutraceutical composition.

In one embodiment, the cosmetic, personal care, pharmaceutical or nutraceutical composition comprises from 2 % to 8 %, from 3 % to 7 % or from 4 % to 6% of dried calcispheres, in weight of the total cosmetic, personal care, pharmaceutical or nutraceutical composition.

In one embodiment, the cosmetic, personal care, pharmaceutical or nutraceutical composition comprises from 0.5 % to 3 % of dried calcispheres, in weight of the total cosmetic, personal care, pharmaceutical or nutraceutical composition. In an alternative embodiment, the obtained cosmetic, personal care, pharmaceutical or nutraceutical composition comprises from 1.0 % to 2.5 % of dried calcispheres, in weight of the total cosmetic, personal care, pharmaceutical or nutraceutical composition.

In one specific embodiment, the recovered microalgae cultured cells are dried without having been subjected to the washing step (c). According to such embodiment, the dried solid phase consists of the whole cells of the *Thoracosphaera heimii* microalgae, that is the organic-matter based cells their calcispheres.

In one embodiment, the cosmetic, personal care, pharmaceutical or nutraceutical composition consists of the dried whole cells as defined above. In one embodiment, the cosmetic, personal care, pharmaceutical or nutraceutical composition comprises from 0.1 % to 99% of the dried whole cells, in weight of the total cosmetic, personal care, pharmaceutical or nutraceutical composition. In one embodiment, the cosmetic, personal care, pharmaceutical or nutraceutical composition comprises from 20 % to 90% of the dried whole cells, in weight of the total cosmetic, personal care, pharmaceutical or nutraceutical composition. In one embodiment, the cosmetic, personal care, pharmaceutical or nutraceutical composition comprises from 30 % to 85% of the dried whole cells, in weight of the total cosmetic, personal care, pharmaceutical or nutraceutical composition. In one embodiment, the cosmetic, personal care, pharmaceutical or nutraceutical composition comprises from 30 % to 70% of the dried whole cells, in weight of the total cosmetic, personal care, pharmaceutical or nutraceutical composition. In one embodiment, the cosmetic, personal care, pharmaceutical or nutraceutical composition comprises from 40 % to 60% of the dried whole cells, in weight of the total cosmetic, personal care, pharmaceutical or nutraceutical composition.

In one embodiment, the cosmetic, personal care, pharmaceutical or nutraceutical composition comprises from 0.1 % to 15% of the dried whole cells, in weight of the total cosmetic, personal care, pharmaceutical or nutraceutical composition. In one embodiment, the obtained cosmetic, personal care, pharmaceutical or nutraceutical composition comprises from 1 % to 10 % of the dried whole cells, in weight of the total cosmetic, personal care, pharmaceutical or nutraceutical composition.

In one embodiment, the cosmetic, personal care, pharmaceutical or nutraceutical composition comprises from 2 % to 8 %, from 3 % to 7 % or from 4 % to 6% of the dried whole cells, in weight of the total cosmetic, personal care, pharmaceutical or nutraceutical composition.

In one embodiment, the cosmetic, personal care, pharmaceutical or nutraceutical composition comprises from 0.5 % to 3 % of the dried whole cells, in weight of the total cosmetic, personal care, pharmaceutical or nutraceutical composition. In an alternative embodiment, the obtained cosmetic, personal care, pharmaceutical or nutraceutical composition comprises from 1.0 % to 2.5 % of the dried whole cells, in weight of the total cosmetic, personal care, pharmaceutical or nutraceutical composition.

The invention further relates to the use of *Thoracosphaera heimii,* as defined in any one of the embodiments above, as a cosmetically, personal care, nutraceutically or pharmaceutically acceptable excipient. The invention further relates to the use of *Thoracosphaera heimii,* as defined in any one of the embodiments above, as a pharmaceutically acceptable excipient in the preparation of a medicament. Typically, the use is as a cosmetically, personal care, or nutraceutically acceptable excipient. In one embodiment, the use is as a cosmetically or personal care excipient.

In one embodiment, any of the above uses is a use as an absorbent. In one embodiment, any of the above uses is a use as a bulking agent. In one embodiment, any of the above uses is a use as an absorbent. In one embodiment, any of the above uses is a use as a carrier of a cosmetically or pharmaceutically active ingredient. In one embodiment, any of the above uses is a use as a carrier of a cosmetically active ingredient.

In one embodiment, the *Thoracosphaera heimii,* as defined in any one of the embodiments above are used as whole cells. Typically, the use of the *Thoracosphaera heimii* refers to the use of the *Thoracosphaera heimii* calcispheres such as for examples the dried calcispheres obtained in step (d) of the present method.

Lastly, the invention relates to the cosmetic, personal care, pharmaceutical or nutraceutical composition, as described in any one of the above embodiments.

In the present invention, unless otherwise specified, any range introduced by "from... to" refers to the range wherein the upper and lower values are included in the range.

### BRIEF DESCRIPTION OF THE DRAWINGS

**Figure 1** shows a microscope photograph of the *T. heimii* cells obtained according to step (b) of the present process
**Figure 2** is a Scanning Electron Microscope photograph showing the *T. heimii* cells obtained in step (b) of the present process. The SEM image was obtained at High Vacuum Mode: 5.0 kV, Magnification 6.00 kx and Working Distance of 6.98 mm.
**Figure 3** shows a Scanning Electron Microscope photograph of a dried *T. heimii* calcisphere obtained in step (d) of the present process (above) and a schematic representation thereof (below). The SEM image was obtained at High Vacuum Mode: 20.0 kV, Magnification 30.00 kx and Working Distance of 7.11 mm.
**Figure 4** shows a Scanning Electron Microscope photograph of a dried *T. heimii* calcisphere structure obtained in step (d) of the present process (above) and a schematic representation thereof (below). The SEM image was obtained at High Vacuum Mode: 5.0 kV, Magnification 18.00 kx and Working Distance of 6.93 mm. The sphere wall thickness D1 is annotated on the SEM image.

### EXAMPLES

The present invention is further illustrated by the following examples. Said examples are provided as illustrative of the invention only and they do not aim at limiting the scope of the invention.

### Example 1: preparation of Thoracosphaera heimii calcispheres

*A Thoracosphaera heimii* was purchased from the Roscoff Culture collection (strain name AC214, RCC1512). *Thoracosphaera heimii* was cultured in an air-lift photobioreactor for three weeks at a temperature below 15°C.

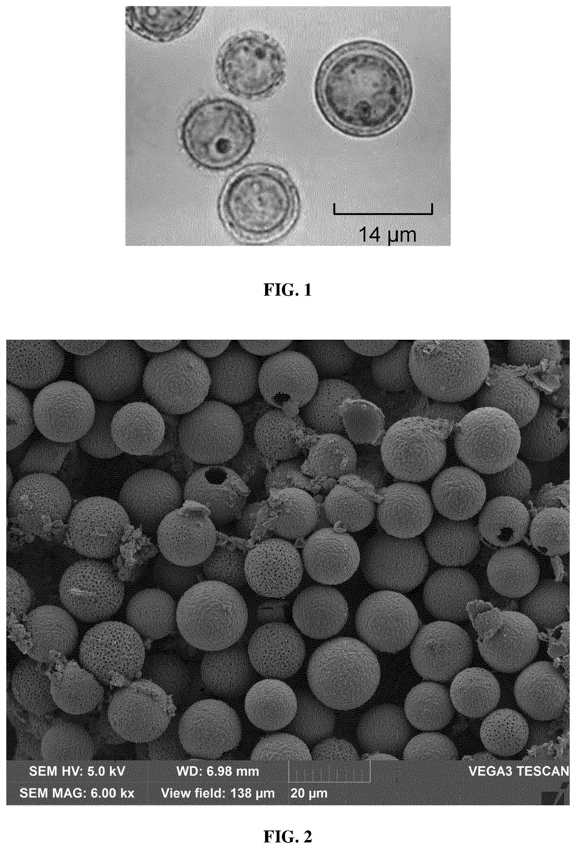

The obtained *Thoracosphaera heimii* presented a homogenous size distribution of about 15 mm mean diameter (Fig. 1). Their structure was analyzed by SEM imaging and presented in Fig.2

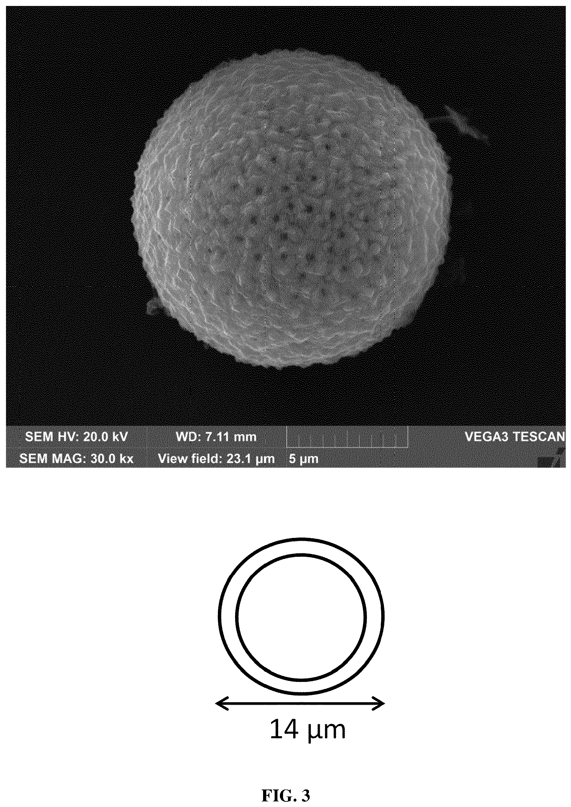

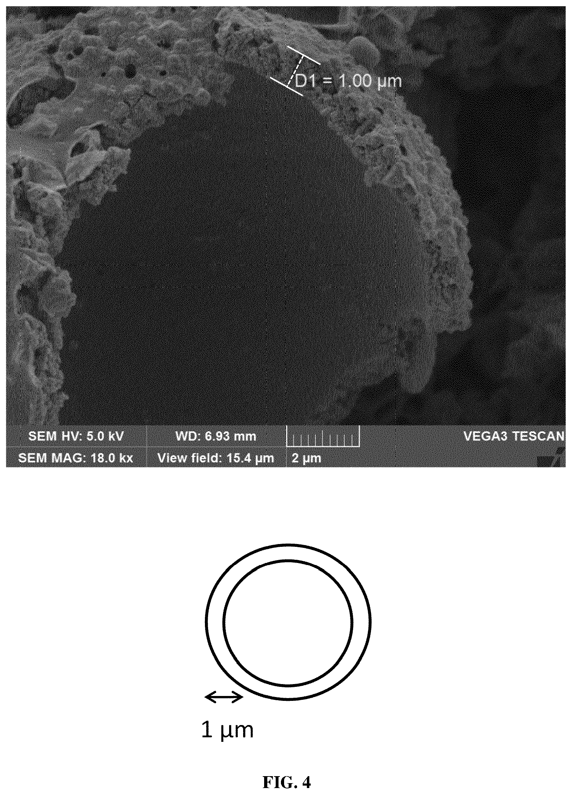

The *T. heimii* cultured cells were recovered and their calcispheres were obtained according to the invention. Their structure was analyzed and presented in Fig. 3 and Fig. 4.

The calcisphere size distribution was assessed by laser scattering using Mastersizer 2000 device from Malvern.

### Example 2: Example of cosmetic composition

A cosmetic composition of a cover foundation has been prepared with the *Thoracosphaera heimii* calcispheres as obtained according to the present description. The composition is presented in the following table 1.

**Table 1 Cosmetic composition according to Example 2.**

| **COVER FOUNDATION** | |
|---|---|
| **Ingredient** | **% in weight from the total weight of the composition** |
| Water | 41% |
| C₁₅-C₁₉ alkane | 16% |
| Olive oil | 10% |
| Polyglyceryl ricinoleate | 8% |
| *T. heimii* calcispheres obtained in example 1 | 5% |
| Mica | 5% |
| Glycerin | 3% |
| Titanium dioxide | 9.1% |
| Iron oxide (yellow) | 2.3% |
| Iron oxide (red) | 0.4% |
| Iron oxide (black) | 0.2% |

## Claims

1. A process for producing a cosmetic, a personal care, a pharmaceutical or a nutraceutical composition, the process comprising:
a) cultivating *Thoracosphaera heimii* microalgae cells, then
b) recovering the cultured microalgae, then
c) washing the recovered *Thoracosphaera heimii* cell culture with an aqueous solution, then rejecting the aqueous solution and recovering solid phase consisting of the *Thoracosphaera heimii* calcispheres, then
d) drying the calcispheres, and then
e) mixing the dried calcispheres of step (d) with at least one cosmetically, personal care, pharmaceutically or nutraceutically acceptable ingredient.

2. The process according to claim **1,** wherein the aqueous solution is 100% water.

3. The process according to claim **1** or claim **2,** wherein the dried calcispheres of step (d) are calcinated prior to the mixing step (e).

4. The process according to any one of claims **1** to **3,** wherein the process is for producing a cosmetic composition and the step (e) consists in mixing the dried calcispheres of step (d) with at least one cosmetically acceptable ingredient.

5. Non-therapeutic use of *Thoracosphaera heimii* microalgae as a cosmetically or nutraceutically acceptable ingredient.

6. The use according to claim **5,** wherein *Thoracosphaera heimii* is used as a whole cell.

7. The use according to claim **5,** wherein *Thoracosphaera heimii* is used as calcispheres.

8. The use according to any one of claims **5** to **7,** wherein *Thoracosphaera heimii* is used as a cosmetically acceptable excipient.

9. The use according to any one of claims **6** to **8,** wherein *Thoracosphaera heimii* is used as a carrier for cosmetically, pharmaceutically or nutraceutically acceptable active ingredient.

10. A cosmetic, personal care, pharmaceutical or nutraceutical composition comprising spherical *Thoracosphaera heimii* calcispheres of less than 15 µm average diameter and of sphere wall thickness of less than 1 µm, in association with at least one cosmetically, pharmaceutically or nutraceutically acceptable ingredient.

11. The cosmetic, personal care, pharmaceutical or nutraceutical composition according to claim **10,** said composition comprising 0.1 % to 100% of the spherical *Thoracosphaera heimii* calcispheres, in weight of the total cosmetic, a personal care, a pharmaceutical or a nutraceutical composition.

## Patentansprüche

1. Verfahren zur Herstellung einer kosmetischen, einer Körperpflege-, einer pharmazeutischen oder einer nutraceutischen Zusammensetzung, wobei das Verfahren umfasst:
a) Kultivieren von *Thoracosphaera heimii*-Mikroalgenzellen, dann
b) Gewinnen der kultivierten Mikroalgen, dann
c) Waschen der gewonnenen *Thoracosphaera heimii*-Zellkultur mit einer wässrigen Lösung, dann Verwerfen der wässrigen Lösung und Gewinnen der festen Phase, die aus den *Thoracosphaera heimii*-Kalksphären besteht, dann
d) Trocknen der Kalksphären, und dann
e) Mischen der getrockneten Kalksphären aus Schritt (d) mit mindestens einem kosmetisch, für die Körperpflege, pharmazeutisch oder nutraceutisch annehmbaren Inhaltsstoff.

2. Verfahren nach Anspruch **1,** wobei die wässrige Lösung zu 100 % Wasser ist.

3. Verfahren nach Anspruch **1** oder Anspruch **2,** wobei die getrockneten Kalksphären aus Schritt (d) vor dem Mischschritt (e) kalziniert werden.

4. Verfahren nach einem der Ansprüche **1** bis **3,** wobei das Verfahren zur Herstellung einer kosmetischen Zusammensetzung dient und der Schritt (e) darin besteht, die getrockneten Kalksphären aus Schritt (d) mit mindestens einem kosmetisch annehmbaren Inhaltsstoff zu mischen.

5. Nichttherapeutische Verwendung von *Thoracosphaera* heimii-Mikroalgen als kosmetisch oder nutraceutisch annehmbarer Inhaltsstoff.

6. Verwendung nach Anspruch **5,** wobei *Thoracosphaera heimii* als Ganzzelle verwendet wird.

7. Verwendung nach Anspruch **5,** wobei *Thoracosphaera heimii* als Kalksphären verwendet wird.

8. Verwendung nach einem der Ansprüche **5** bis **7,** wobei *Thoracosphaera heimii als* kosmetisch annehmbarer Hilfsstoff verwendet wird.

9. Verwendung nach einem der Ansprüche **6** bis **8,** wobei *Thoracosphaera heimii* als Träger für einen kosmetisch, pharmazeutisch oder nutraceutisch annehmbaren Wirkstoff verwendet wird.

10. Kosmetische, Körperpflege-, pharmazeutische oder nutraceutische Zusammensetzung, umfassend sphärische *Thoracosphaera* heimii-Kalksphären mit einem mittleren Durchmesser von weniger als 15 µm und einer Sphärenwanddicke von weniger als 1 µm, in Verbindung mit mindestens einem kosmetisch, pharmazeutisch oder nutraceutisch annehmbaren Inhaltsstoff.

11. Kosmetische, Körperpflege-, pharmazeutische oder nutraceutische Zusammensetzung nach Anspruch **10,** wobei die Zusammensetzung 0,1 % bis 100 % der sphärischen *Thoracosphaera* heimii-Kalksphären, bezogen auf das Gewicht der gesamten kosmetischen, Körperpflege-, pharmazeutischen oder nutraceutischen Zusammensetzung, umfasst.

## Revendications

1. Procédé de production d'une composition cosmétique, de soin personnel, pharmaceutique ou nutraceutique, le procédé comprenant :
a) la culture de cellules de microalgues *Thoracosphaera heimii,* puis
b) la récupération des microalgues cultivées, puis
c) le lavage de la culture cellulaire de *Thoracosphaera heimii* récupérée avec une solution aqueuse, puis le rejet de la solution aqueuse et la récupération d'une phase solide constituée de calcisphères de *Thoracosphaera heimii,* puis
d) le séchage des calcisphères, et puis
e) le mélange des calcisphères séchées de l'étape (d) avec au moins un ingrédient cosmétiquement, de soin personnel, pharmaceutiquement ou nutraceutiquement acceptable.

2. Le procédé selon la revendication 1, dans lequel la solution aqueuse est à 100 % d'eau.

3. Le procédé selon la revendication **1** ou la revendication **2,** dans lequel les calcisphères séchées de l'étape (d) sont calcinées avant l'étape de mélange (e).

4. Le procédé selon l'une quelconque des revendications **1** à **3,** dans lequel le procédé est pour la production d'une composition cosmétique et l'étape (e) consiste à mélanger les calcisphères séchées de l'étape (d) avec au moins un ingrédient cosmétiquement acceptable.

5. Utilisation non thérapeutique de microalgues *Thoracosphaera heimii* en tant qu'ingrédient cosmétiquement ou nutraceutiquement acceptable.

6. L'utilisation selon la revendication **5,** dans laquelle *Thoracosphaera heimii* est utilisée sous forme de cellule entière.

7. L'utilisation selon la revendication **5,** dans laquelle *Thoracosphaera heimii* est utilisée sous forme de calcisphères.

8. L'utilisation selon l'une quelconque des revendications **5** à **7,** dans laquelle *Thoracosphaera heimii* est utilisée en tant qu'excipient cosmétiquement acceptable.

9. L'utilisation selon l'une quelconque des revendications **6** à **8,** dans laquelle *Thoracosphaera heimii* est utilisée comme vecteur pour un principe actif cosmétiquement, pharmaceutiquement ou nutraceutiquement acceptable.

10. Composition cosmétique, de soin personnel, pharmaceutique ou nutraceutique comprenant des calcisphères sphériques de *Thoracosphaera heimii* ayant un diamètre moyen inférieur à 15 µm et une épaisseur de paroi de sphère inférieure à 1 µm, en association avec au moins un ingrédient cosmétiquement, pharmaceutiquement ou nutraceutiquement acceptable.

11. La composition cosmétique, de soin personnel, pharmaceutique ou nutraceutique selon la revendication **10,** ladite composition comprenant de 0,1 % à 100 % de calcisphères de *Thoracosphaera heimii* sphériques, en poids par rapport au poids total de la composition cosmétique, de soin personnel, pharmaceutique ou nutraceutique.
